# EUROPEAN PATENT APPLICATION

(11) **EP 1 992 637 A1**
(43) Date of publication of application: **19.11.2008**
(21) Application number: 07290629.0
(22) Date of filing: 16.05.2007
(51) Int. Cl.: C07K 5/06, C07K 1/02, A61K 38/05

(54) **1-Aminocyclopentane-1,3,4- tricarboxylic acids derivatives**

(71) Applicant: Faust Pharmaceuticals, 67400 Illkirch (FR)
(72) Inventor: Schann, Stephan, 67118 Geispolsheim (FR); Mayer, Stanislas, 67114 Eschau (FR)

(57) **Abstract**

The invention provides derivatives of 1-aminocyclopentane-1,3,4-tricarboxylic acids which provide a sufficient bioavailability upon oral administration to be conveniently used as medicaments modulating the effect of glutamate.

## Description

The invention relates to compounds which are effectors of central nervous system receptors sensitive to neuroexcitatory amino acids such as glutamate. In particular, the invention provides derivatives of 1-aminocyclopentane-1,3,4-tricarboxylic acids which provide a sufficient bioavailability upon oral administration to be conveniently used as medicaments modulating the effect of glutamate.

It is known that glutamate is involved in numerous cerebral functions. Important roles are therefore attributed to glutamatergic receptors, in particular as regards the conduction of nerve impulse, synaptic plasticity, the development of the central nervous system, learning and memory.
Glutamate is also the main endogenous neurotoxin, being responsible for the neuronal death observed after ischemia, hypoxia, epileptic fits or traumatisms of the brain. It is not therefore surprising that glutamate receptors are considered to be involved in various disorders of the central nervous system and neurodegenerative diseases.
Two main types of glutamatergic receptors have been characterized: ionotropic receptors and metabotropic receptors. The ionotropic receptors are cationic channels activated by the glutamate and directly responsible for the rapid depolarization of post-synaptic cells. They are compounds of different sub-units and classified into three groups according to their pharmacological and functional properties. A distinction is thus made between the NMDA receptors (N-methyl-D-aspartic acid), AMPA (alpha-amino-3-hydroxy-5-methyl-isoxazole-4-propionic acid) and kainate. Metabotropic receptors (mGluRs) have been revealed in the mid eighties (1985). They have been found to play an important role in particular in the induction of the long-term potentialization (LTP) and the long-term depression (LDP) of synaptic transmission, in the regulation of baroceptive reflexes, spatial learning, motor learning, postural and kinetic integration, and are likely involved in acute or chronic degenerative diseases such as epilepsy, Alzheimer's disease, Parkinson's disease or Huntington chorea as well as neuropsychiatric disorders such as anxiety, depression and schizophrenia.

A new class of ligands of metabotropic glutamate receptors is disclosed in WO 98/43907 and the corresponding US-Patent No. 6,433,014. Among the cyclic analogues of glutamic acid disclosed in these documents, 1-aminocyclopentane-1,3,4-tricarboxylic acids (ACPTs) have been found to be of great interest. In particular, the compound identified as ACPT-I or meso-cis ACPT ((1S, 3R, 4S) -1-aminocyclopentane-1,3,4-tricarboxylic acid) has shown to be efficient as a selective group III metabotropic glutamate receptor agonist with a high potential as an active agent for modulating the effect of glutamate.
However, 1-aminocyclopentane-1,3,4-tricarboxylic acid shows a low oral bioavailability, which makes it difficult to effectively provide oral dosage forms of the compound.

### BRIEF SUMMARY OF THE INVENTION

In view of the above, it is the aim of the invention to provide derivatives of 1-aminocyclopentane-1,3,4-tricarboxylic acid as prodrugs which have an improved oral bioavailability while retaining the pharmaceutical effectiveness of their parent compound. Moreover, it is a further aim of the invention to provide pharmaceutical compositions, in particular pharmaceutical compositions for oral or nasal administration, which contain the derivatives according to the invention. Still further, the invention aims at the provision of methods for the treatment of conditions requiring a modulation of the effect of glutamate in a patient, comprising the administration of such pharmaceutical compositions.

In particular, the invention thus provides the compounds of formula (I) 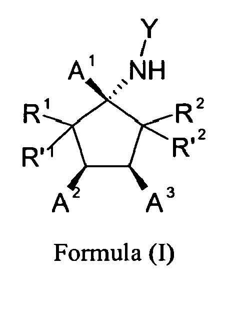 wherein
R¹, R'¹, R² and R'² are independently selected from hydrogen, optionally substituted alkyl, aryl, fluorine or hydroxy;
A¹, A² and A³ are independently selected from -COOH, its salts, esters or amides;
Y represents a -(W)ₙ-H group with
W represents an amino-acyl group corresponding to natural or non-natural amino-acids;
and
n represents an integer comprised between 1 and 10.

It will be appreciated that compounds of formula (I) contain at least three asymmetric carbons. The present invention includes all stereoisomeric forms of the compounds.

### BRIEF DESCRIPTION OF THE DRAWINGS

Figures 1 illustrates parent compound (ACPT-I) plasma exposure obtained after oral administration to rats of prodrugs of the invention (examples 1, 3 and 4) and ACPT-I at 30 mg/kg and intravenous administration of the parent compound at dose of 10 mg/kg.
Figures 2 shows the results of ACPT-I evaluation in a mouse model of Parkinson disease, the haloperidol-induced catalepsy, after oral and intraperitoneal administrations at doses of 100 mg/kg and 30 mg/kg respectively.
Figure 3 shows the results of examples 1 and 3 evaluations in a mouse model of Parkinson disease, the haloperidol-induced catalepsy, after oral administration at 100 mg/kg.

### DETAILED DESCRIPTION OF THE INVENTION

The term "alkyl" as used herein, unless indicated otherwise, refers to a respective group preferably having 1 to 20 carbon atoms, more preferably 1 to 10 and particularly preferably 1 to 8 or 1 to 6 carbon atoms. Such alkyl groups may be linear, branched or cyclic and include methyl, ethyl, propyl, butyl, pentyl and hexyl groups. An alkyl group may be unsubstituted or may carry one ore more substituents, which may be selected, e.g., from hydroxyl, halogen and aryl.

The term "aryl" as used herein, unless indicated otherwise, refers to a respective group preferably having 6 to 18 carbon atoms, particularly 6 to 10 carbon atoms. It includes groups such as a phenyl or naphtyl group. An aryl group may be unsubstituted or may carry one ore more substituents, which may be selected, e.g., from alkyl, hydroxyl, halogen, O-alkyl and nitro.

The term "halogen" as used herein, unless indicated otherwise, includes atoms such as fluorine, chlorine, bromine or iodine.

In the compounds of formula (I), A¹, A² and A³ may be the same or different and are independently selected from a carboxylic acid group and a salt, ester or amide thereof. Preferably, A² and A³ are identical. Particularly preferred is the case where A¹, A² and A³ are identical.
Carboxylic acid salts as A¹, A² and/or A³ may be formed with any pharmaceutically acceptable inorganic or organic cation. Exemplary inorganic cations include alkali metal cations such as sodium and potassium ions and ammonium ions. Exemplary organic cations include ammonium ions substituted by one, two, three or four alkyl groups.

A salt of an amino function of compounds of formula (I) may be formed with any pharmaceutically acceptable inorganic or organic anion. Exemplary inorganic anions include chloride, bromide, nitrate, carbonate, monohydrogenocarbonate, phosphate, monohydrogenophosphate, dihydrogenophosphate and the like. Exemplary organic anions include formate, acetate, lactate, propionate, butyrate, isobutyrate, palmoate, glutamate, maleate, malonate, benzoate, succinate, fumarate, salicylate, citrate, tartarate, methanesulfonate, phenylsulfonate and the like.
Ester groups of a carboxylic acid as A¹, A² and/or A³ preferably have the structure -COOR⁵, wherein R⁵ is an alkyl or aryl group. Preferred as R⁵ is a C1-C4 alkyl group, such as methyl, ethyl, propyl or butyl, and particularly preferred is an unsubstituted C1-C4 alkyl group.
Amide groups of a carboxylic acid as A¹, A² and/or A³ preferably have the structure -CONR⁶R⁷, wherein R⁶ and R⁷ are independently selected from hydrogen, alkyl or aryl. Particularly preferably, R⁶ and R⁷ are selected from hydrogen and from a C1-C4 alkyl group, such as methyl, ethyl, propyl or butyl, and particularly preferred from hydrogen and an unsubstituted C1-C4 alkyl group.
Preferably, at least two of the groups A¹, A² and A³ are carboxylic acid groups. More preferably, all of A¹, A² and A³ are carboxylic acid groups.

In the compounds of formula (I), R¹, R'¹, R² and R'² are independently selected from hydrogen, alkyl, aryl, fluorine or hydroxy. Preferred as R¹, R'¹, R² and R'² are hydrogen and fluorine and particularly preferred is hydrogen.

In the compounds of formula (I), W is an amino-acyl group -C(O)-L-NH- wherein the structure of L is defined such that the amino acyl group corresponds to a natural or non-natural amino-acid and wherein the carbonyl group of W or of the first of a chain of n groups W forms an amide bond with the amino function of the compound of formula (I) to which Y is bound. An amino-acid is a molecule that contains at least one amine function and one carboxylic acid function.
In cases where W is an amino-acyl group corresponding to one of the twenty natural amino-acids, such natural amino-acid could be glycine (Gly), leucine (L-Leu), phenylalanine (L-Phe), alanine (L-Ala), valine (L-Val), isoleucine (L-Ile), histidine (L-His), aspartic acid (L-Asp), glutamic acid (L-Glu), asparagine (L-Asn), glutamine (L-Gln), arginine (L-Arg), tyrosine (L-Tyr), cysteine (L-Cys), methionine (L-Met), lysine (L-Lys), serine (L-Ser), threonine (L-Thr), tryptophan (L-Trp), and proline (L-Pro).
In cases where W is an amino-acyl corresponding to a non natural amino-acid, such amino-acid could be the D-isomers of the natural amino-acids indicated above, beta-alanine (β-Ala), allylglycine, alloisoleucine (αIle), norvaline (Nva), norleucine (Nle), neopentylglycine, 2-amino-3,3-dimethylbutyric acid (tert-Leu), citrulline (Cit), cystathionine, cysteic acid, L-DOPA, homocysteine, homoserine, lanthionine, ornithine, 5-oxoproline, selenocysteine, thyronine, thyroxine, 2-aminobutyric acid (Abu), 2,4-diaminobutyric acid (A₂bu), 2-aminoisobutyric acid (β-Aib), 3-aminobutyric acid, 4-aminobutyric acid (GABA), 2-aminohexanoic acid (Ahx), 6-aminohexanoic acid (εAhx), 2-aminoadipic acid (Aad), 3-aminoadipic acid (βAad), 2,3-diaminopropionic acid (A₂pr), ornithine (Orn), 2-aminopimelic acid (Apm), 2,6-diaminopimelic acid (A₂pm), 2,3-diaminopropionic acid (Dpr), 2-aminotetradecanoic acid, 3-(2-naphtyl)alanine (2-Nal), 3-(1-naphtyl)alanine (1-Nal), β-cyclopropyl-alanine, β-cyclohexyl-alanine (Cha), 1-amino-cyclohexane acetic acid, 1-aminomethyl-cyclohexane acetic acid (Gabapentin), 3-(aminomethyl)-5-methylhexanoic acid (pregabalin), baclofen, saclofen, vigabatrin, LY354740, LY404039, ACPT-I, (+)-ACPT-III, 2-aminoisobutyric acid, 1-amino-1-cyclopropane carboxylic acid, 1-amino-1-cyclobutane carboxylic acid, 1-amino-1-cyclopentane carboxylic acid, 1-amino-1-cyclohexane carboxylic acid, hydroxyproline, sarcosine (Sar), O-methyl-tyrosine, phenyl glycine and the like; and amino-acids in which the α-carbon bears two side-chain substituents; and cyclic amino-acids.
In cases where W is an amino-acyl corresponding to a non natural amino-acid, such amino-acid could be a compound of formula (I) with R¹, R'¹, R², R'², A¹, A², A³ as defined for formula (I) and Y is a H atom.
In a preferred embodiment the W group of formula (I) is an amino-acyl group corresponding to a natural amino-acid.

More preferably is the case where the W group corresponds to a neutral natural amino-acid selected from L-Leu, L-Ala, L-Val, L-Ile, L-Phe, Gly, L-Met, L-Ser, L-Asn, L-Gln, L-Tyr, L-Cys, L-Thr, L-Trp and L-Pro.
More preferably is the case where W group corresponds to a neutral amino-acid selected from L-Leu, L-Ala, L-Val, L-Ile and L-Phe.

In the compounds of formula (I), n is an integer between 1 and 10. Preferably between 1 and 5 and more preferably n is 1 or 2 (each range including the upper and lower limit). In cases where n is an integer of 2 or more, the groups W may be identical or different and are generally linked to each other via amide bonds to form an oligopeptide structure.

The names of natural and non-natural amino acids used herein follow the naming conventions suggested by the IUPAC-IUB Joint Commission on Biochemical Nomenclature (JCBN) as set out in "Nomenclature and Symbolism for Amino Acids and Peptides" (Recommendations, 1983) European Journal of Biochemistry, 138, 9-37 (1984).

The compounds of formula (I) may be conveniently prepared by reacting the parent compounds of formula (II) 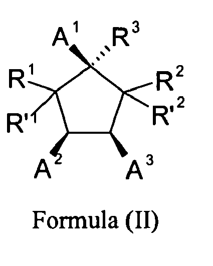 wherein R¹, R'¹, R², R'², A¹, A² and A³ are as defined for the compounds of formula (I) and R³ represents an amino group, with a natural or a non natural amino-acid or a peptide under conditions where an amide bond is formed between the reactants.

Compounds of formula (II) may be obtained, e.g., following the method described by Acher et al., J. Med. Chem. 1997, 40, 3119-3129 or in WO 98/34907. Alternatively, a very efficient reaction to obtain a 1-aminocyclopentane-1,3,4-tricarboxylic acid or its derivatives as defined by formula (II) above is the hydrogenation of a compound of formula (III) 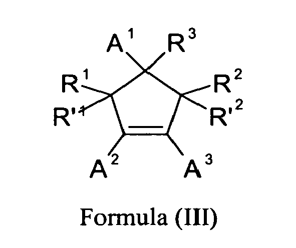 wherein R¹, R'¹, R², R'², A¹, A² and A³ are as defined for the compounds of formula (I) and R³ represents an amino group or its protected form in the presence of a catalyst. The hydrogenation proceeds with a high selectivity to yield the compounds of formula (II). Suitable catalysts can be chosen, e.g., from Pt or Pd supported on active carbon (Pt/C or Pd/C) or other forms of elementary platinum or palladium, Raney nickel, PtO₂, nickel boride, rhodium, ruthenium, Wilkinson's catalyst or Crabtree's catalyst, among which further preference is given to PtO₂ and Pd/C.

A particularly beneficial reaction sequence to obtain two preferred compounds of formula (II) (referred to as intermediate 4 and ACPT-I in this scheme) is shown in the following:

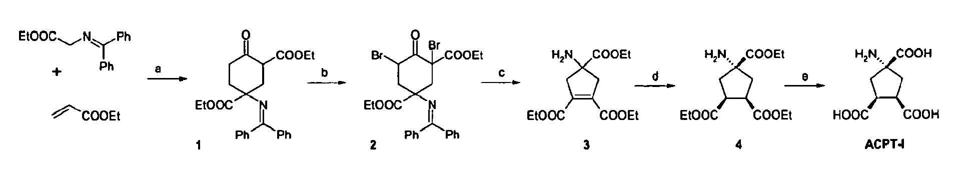

In step (a), ring formation is effected in the presence of a base to yield intermediate 1. In step (b), compound 1 is brominated to yield intermediate 2. In step (c), the Favorskii rearrangement forms a compound having a cyclopentene skeleton under basic conditions, followed by deprotection of the amino group to yield intermediate 3. In step (d), this intermediate is hydrogenated in the presence of H₂ and a catalyst to yield compound 4 as the only stereoisomer. In step (e), the ester groups may be removed to yield the free 1-aminocyclopentane-1,3,4-tricarboxylic acid, referred to as ACPT-1 in the above scheme.

For the preparation of compounds of formula (I), intermediate 4 can be reacted with an appropriate amino-acid or peptide to give the corresponding peptide that can be further saponified as shown in the examples below. Alternatively, compounds of formula (I) can be prepared by silylation of ACPT-I followed with coupling of the intermediate with an appropriate amino-acid or peptide and final deprotection following similar conditions as the ones described by Coffey DS et al in Tetrahedron Letters 46 (2005) 7299-7302.

Alternatively, compounds of formula (I) can be prepared by reaction of cyclopentene 3, a preferred compound a formula (III), with an amino-acid or peptide compounds followed by catalytic hydrogenation and deprotections.

During the reaction of the compound of formula (II) above with an amino-acid or peptide, the amino group(s) present in the amino-acid or peptide is (are) preferably protected by a protective group known in the art (as described, e.g. Green, T;W.; Wuts, P.G.M. Protective Groups in Organic Synthesis, 3nd ed.; Wiley, New York, 1999, chapter 7, page 494), such as a t-butyl carbamate (boc) group. This protective group may be removed after the amide formation has taken place by methods well established in the art.

It should be understood that, based on common general knowledge, the skilled person can choose a variety of other synthetic routes to obtain the compounds according to the invention.

The compounds according to the invention represent prodrugs of effectors of metabotropic glutamate receptors of the ACPT family which provide a suitable bioavailability in particular after oral or nasal administration. As such, they can be conveniently used as medicaments for the treatment of conditions where a modulation the effect of glutamate is required or useful.

Such conditions associated with altered glutamatergic signaling and/or functions, and/or conditions which can be affected by alteration of glutamate level or signalling are selected from, but not limited to epilepsy, dementias (including dementias of the Alzheimer's type, vascular dementias, AIDS-dementia complex), parkinsonism and movement disorders (including Huntington's disease, dystonias, Gilles de la Tourette syndrome, dyskinesias), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis), headache, migraine, myoneural disorders (myasthenia gravis, acute muscle spasms, myopathies), disorders of the eye and visual pathways, intracranial trauma/injury, trauma/injury to nerves and spinal cord, poisoning and toxic effects of nonmedicinal substances, accidental poisoning by drugs, medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including attention deficit and disruptive behaviour disorders, autism, TIC disorders), delirium and other cognitive disorders, substance related disorders (caused e.g. by alcohol, nicotine, drugs), schizophrenia and other psychotic disorders, mood disorders (including depressive disorders and bipolar disorders), anxiety disorders, sexual disorders, eating disorders, sleep disorders, endocrine and metabolic diseases (diabetes, hypoglycaemia), acute and chronic pain; nausea and vomiting; irritable bowel syndrome.

Methods of treatment which are encompassed by the present invention comprise administering a safe and effective amount of at least one compound selected from the compounds of formula (I), or a pharmaceutical composition containing at least one compound selected from the compounds of formula (I), to a patient in need thereof. As used herein, "patient" refers to a human or other animal.
The term "treatment" as used herein relates to the amelioration or prevention of the condition being treated or of one or more of the biological symptoms of the condition being treated.
A safe and effective amount of a compound of the invention will vary with the particular compound chosen (e.g. depending on prodrug ability to deliver the active parent molecule in the body); the route of administration chosen; the condition being treated; the severity of the condition being treated; the age, size, weight, and physical condition of the patient being treated; the duration of the treatment and like factors. It can be routinely determined by the skilled practitioner. Typical daily dosages may vary depending upon the particular route of administration chosen and range from about 1 to about 1000 mg/kg.
The compounds of the invention may be administered by any suitable route of administration, including systemic administration and topical administration. Systemic administration includes oral administration, parenteral administration, transdermal administration, rectal administration or inhalation. Parenteral administration refers to routes of administration other than enteral or transdermal, and is typically by injection or infusion. Parenteral administration includes intravenous, intramuscular, and subcutaneous injection or infusion. Topical administration includes application to the skin as well as intraocular, optic, intravaginal, and intranasal administration. In view of the beneficial bioavailability of the compounds according to the invention via the oral route, oral administration is preferred. This includes the administration via the mouth or the nose.
The compounds of the invention may be administered once or in doses at varying intervals of time for a given period of time. For example, doses may be administered one, two, three, or four times per day. Suitable dosage regimens for a compound of the invention can be routinely determined by the skilled practitioner.

The compounds of formula (I) may be used in combination with other active agents.
The compounds of formula (I) will usually, but not necessarily, be formulated into pharmaceutical compositions prior to administration to a patient. The invention thus encompasses pharmaceutical compositions comprising a compound of the invention, typically together with one or more pharmaceutically acceptable excipients and/or carriers.
The pharmaceutical compositions of the invention may be prepared and packaged in bulk form or in unit dosage forms. When provided in unit dosage form, the pharmaceutical compositions of the invention typically contain from about 1 mg to about 2000 mg.
The compound of the invention and the pharmaceutically acceptable excipient or excipients will typically be formulated into a dosage form adapted for administration to the patient by the desired route of administration. Dosage forms adapted for oral administration include tablets, capsules, pills, troches, powders, syrups, elixirs, suspensions, solutions, emulsions, and sachets.
Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen in order to facilitate the production of uniform dosage forms. Certain pharmaceutically acceptable excipients may be chosen for their ability to stabilize the compound or compounds of the invention in a pharmaceutical composition. Certain pharmaceutically acceptable excipients may be chosen for their ability to enhance patient compliance.
Suitable pharmaceutically acceptable excipients include binders, lubricants, glidants,disintegrants, granulating agents, coating agents, wetting agents, solvents, cosolvents, suspending agents, flavoring agents, flavor masking agents, anticaking agents, humectants, chelating agents, plasticizers, viscosity regulating agents, antioxidants, preservatives, stabilizers, surfactants, emulsifiers, and buffering agents. The pharmaceutical compositions of the invention are prepared using techniques and methods known to those skilled in the art, e.g. as described in Remington's Pharmaceutical Sciences (Mack Publishing Company).

Oral dosage forms may comprise a carrier in the form of a diluent or filler. Suitable diluents and fillers in general include lactose, sucrose, dextrose, sorbitol, mannitol, cellulose or cellulose derivatives such as microcrystalline cellulose, starch, calcium sulfate, and dibasic calcium phosphate. A liquid dosage form will generally consist of a suspension or solution of the compound or salt in a liquid carrier for example, ethanol, olive oil, glycerine, glucose syrup or water. Where the composition is in the form of a tablet or lozenge, any pharmaceutical carrier routinely used for preparing solid formulations may be used, such as magnesium stearate, terra alba, talc, gelatin, acacia, stearic acid, starch, lactose and sucrose. Where the composition is in the form of a capsule, any routine encapsulation is suitable. Where the composition is in the form of a soft shell capsule e.g. gelatin, any pharmaceutical carrier routinely used for preparing dispersions or suspensions may be incorporated, for example aqueous gums or oils. Preparations may be suitably formulated to give controlled/extended release of the active compound.
An oral solid dosage form may further comprise an excipient in the form of a binder. Suitable binders include starch, gelatin, alginic acid, sodium alginate, tragacanth, acacia, guar gum, povidone, and cellulose or cellulose derivatives such as microcrystalline cellulose. The oral solid dosage form may further comprise an excipient in the form of a disintegrant. Suitable disintegrants include crospovidone, sodium starch glycolate, croscarmelose, alginic acid, and sodium carboxymethyl cellulose. The oral solid dosage form may further comprise an excipient in the form of a lubricant. Suitable lubricants include stearic acid, magnesium stearate, calcium stearate, and talc.

### EXAMPLES

### General:

All reagents were commercial grade and used without further purification. Commercially available anhydrous solvents were used for reactions conducted under inert atmosphere. Silica gel generally used for column chromatography was SDS silica gel (60AAC 40-63 µM). Thin layer chromatography was carried out using pre-coated silica gel F-254plate. ¹H NMR spectra were recorded on a Bruker 300 or 400 spectrometer. Proton chemical shifts are listed relative to residual CDCl₃ (7.27 ppm) or D₂O (4.60 ppm). Splitting patterns are designated as s (singlet), d (doublet), dd (double-doublet), t (triplet), q (quartet), m (multiplet), br (broad).
Electrospray MS spectra were obtained on a Waters micromass platform LCMS spectrometer.
All mass spectra were full-scan experiments (mass range 100-800 amu). Mass spectra were obtained using an electro spray ionization. The HPLC system was a Waters platform with a 2767 sample manager, an 2525 pump, a photodiode array detector (190-400 nM). The column used was an Xterra C₁₈ 3.5 µM (4.6 x 50 mm) in analytical mode and an Xterra C18 OBD 5 µM (30 x 100 mm) in preparative mode. The mobile phase in both cases consisted in an appropriate gradient of A and B. A was water with 0.05 % of TFA and B was acetonitrile with 0.05 % of TFA. Flow rate was 1 mL per min. in analytical mode and 25 mL min in preparative mode. All LCMS were performed at room temperature.
In preparative mode two conditions were set-up.
Condition A: 100 % A for 5 minutes, then from 100 % A to 90 % A in 10 minutes.
Condition B: 95 % A for 5 minutes, then from 95 % A to 80 % A in 10 minutes.

### 1) Preparation of Reactants

### Preparation of (1S, 3R, 4S) -1-aminocyclopentane-1,3,4-tricarboxylic acid triethyl ester

### Step one:

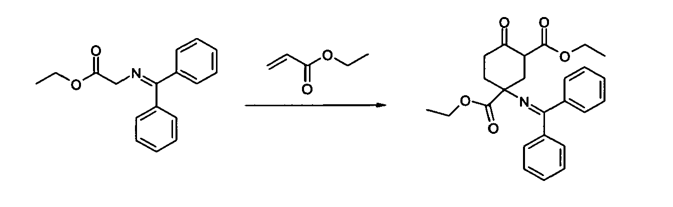

To a stirred solution of Bu₄NBr (3.0 g) and sodium ethoxide (31.8 g) in acetonitrile (200 mL) cooled by an ice bath, under a nitrogen atmosphere, a solution of ethyl N-(diphenylmethylene)glycinate (25.0 g) and ethyl acrylate (51.0 g) in acetonitrile (200 mL) was added. The mixture was stirred at 0°C to 4°C overnight then 4 Hrs at 5°C to 10°C and then was filtered. The filtrate was hydrolyzed with 5% citric acid (aq.) and extracted with ethyl acetate. The organic layer was dried and solvent removed to give an orange oil which was purified by chromatography on silica gel (ethyl acetate : isohexane (10 :90)) to give a mixture of cyclohexanone diastereomers as a yellow oil (30.0 g, 76%).
M/Z (M+H₂O-benzophenone+H)⁺ = 258.

### Step two:

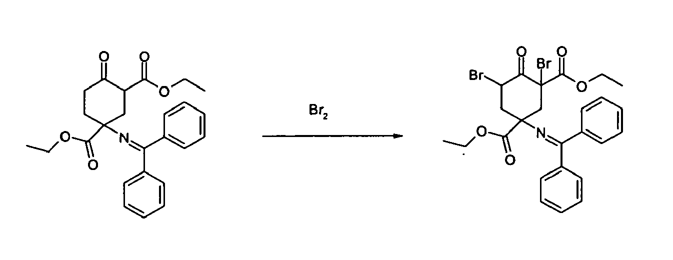

To a solution of cyclohexanone intermediate obtained in step 1 (29.2 g) in CHCl₃ (150 mL), cooled to 0°C, bromine (1 M in CHCl₃ - 138.5mL) was slowly added over 20 min. The reaction mixture was stirred at 0°C for 1.5 Hrs, then was allowed to warm to room temperature overnight. The solution was poured onto a mixture of ice and saturated sodium bicarbonate (aq.) then was extracted with DCM. The organic layer was washed with brine, dried over MgSO₄ and then evaporated to give a yellow oil which was purified by chromatography on silica gel (ethyl acetate : isohexane (20 : 80)) to give the dibromo derivative (28.0 g, 70%).
M/Z (M+H)⁺ = 580.

### Step three:

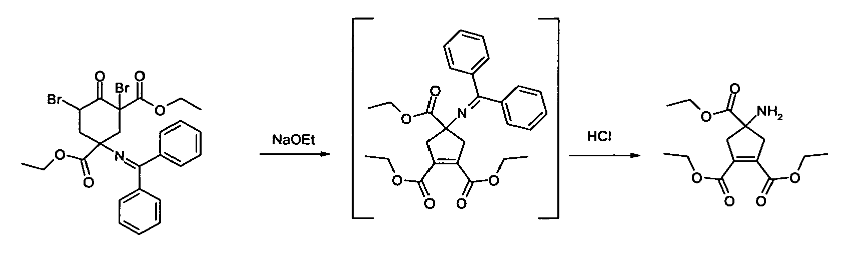

To a solution of sodium ethoxyde (6.7 g) in dry ethanol (300 mL) at 0°C, the dibromo material obtained in step 2 (26.0 g) in solution in dry ethanol (300 mL) was added. The reaction mixture was stirred at 0°C for 1 Hrs, then was poured into aqueous 1M HCI (150 mL) at 0°C. The reaction mixture turned cloudy, and was stirred 30 minutes below 10°C. The clear solution was extracted with diethyl ether. The aqueous layer was neutralized with saturated NaHCO₃ and extracted with ethyl acetate. The ethyl acetate layer was dried over Na₂SO₄, concentrated and purified by chromatography on silica gel (ethyl acetate : isohexane (60 :40)) to give the cyclopentene amino-ester (2.9 g, 22%).
¹H-NMR (300 MHz, CDCl₃): 1.28 (m, 9H), 2.66 (d, 2H, J=15.4 Hz), 3.35 (d, 2H, J=15.4 Hz), 4.22 (m, 6H).

### Step four:

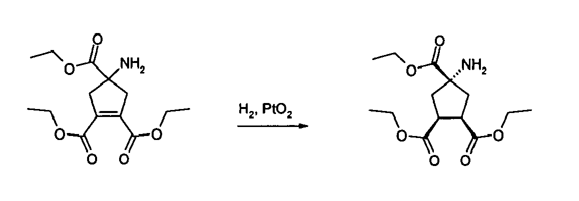

To a solution of cyclopentene derivative obtained in step 3 (1.5 g) in ethanol (90 mL), PtO₂ (60 mg) was added. The reaction mixture was purged with hydrogen, and a pressure of 1 atm of hydrogen was maintained overnight. The suspension was filtered and the filtrate was concentrated to give (1S, 3R, 4S) -1-aminocyclopentane-1,3,4-tricarboxylic acid triethyl ester as a light brown oil (1.5 g, 99%).
¹H-NMR (300 MHz, CDCl₃): 1.24 (m, 9H), 1.95 (m, 2H), 2.54 (m, 2H), 3.46 (m, 2H), 4.15 (m, 6H).

### 2) Preparation of the compounds according to the invention

### Example 1:

### (1S, 3R, 4S)-1-{[(2'S)-2'-amino-4'-methylpentanoyl]amino}cyclopentane-1,3,4-tricarboxylic acid (L-Leu A CPT-I):

Step 1:

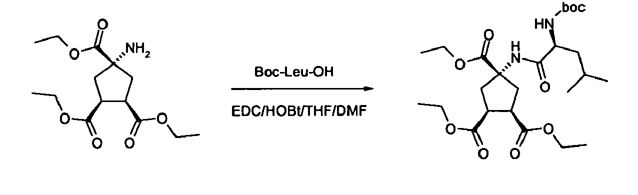

To a solution of (1S, 3R, 4S) -1-Aminocyclopentane-1,3,4-tricarboxylic acid triethyl ester (920 mg), Boc-Leu-OH (774 mg) and HOBt (452 mg) in THF/DMF, cooled to 0°C, EDC (641 mg) was added portionwise. The reaction mixture was allowed to warm to room temperature overnight. The reaction mixture was hydrolyzed with a cold citric acid solution and was extracted with ethyl acetate. The organic layer was washed with saturated NaHCO₃, with brine, was dried over MgSO₄, then was concentrated. The crude product was purified by chromatography on silica gel (ethyl acetate : hexane (1 : 3)) to give the protected L-Leu-ACPT-I as a yellow oil (1.4 g, 89 %).
¹H-NMR (300 MHz, CDCl₃): 0.94 (dd, 6H, J=6.3, 5.9 Hz), 1.24 (m, 9H), 1.45 (s, 9H), 1.65 (m, 3H), 2.41 (m, 2H), 2.71 (m, 2H), 3.39 (m, 2H), 4.01 (m, 1H), 4.14 (m, 6H), 4.82 (brd, 1H, J=7.9 Hz), 6.86 (bs, 1H).

### Step 2:

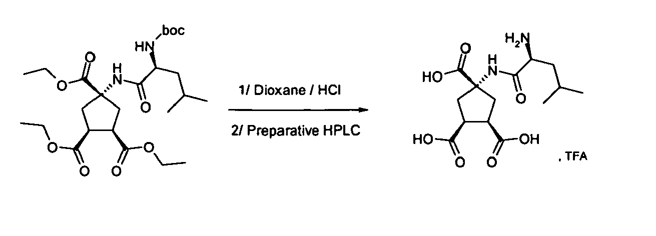

The protected L-Leu-ACPT-I (373 mg) in solution in dioxane : 2M HCl mixture (1:1 ratio - 7.3 mL) was heated at 80°C for 24 Hrs. The reaction mixture was cooled, solvent volume was reduced to a minimum and the product was isolated using prep HPLC (condition B) to afford the trifluoroacetic acid salt of the L-Leu-ACPT-I as a white solid (120 mg, 50%).
¹H-NMR (400 MHz, D₂O): 0.89 (dd, 6H, J=6.4, 6.4 Hz), 1.65 (m, 3H), 2.38 (m, 2H), 2.62 (m, 2H), 3.34 (m, 2H), 3.93 (m, 1H).
M/Z (M+H)⁺= 331.

### Example 2:

### (1S, 3R, 4S)-1-{[(2'S)-2'-amino-3'phenylpropanoyl]amino}cyclopentane-1,3,4-tricarboxylic acid (L-Phe-ACPT-I):

### Step 1

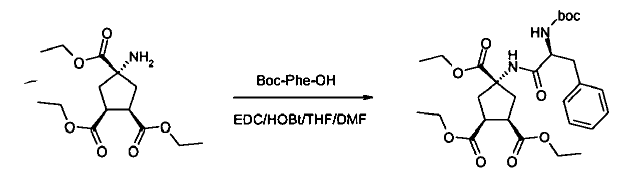

The L-Phe-ACPT-I was obtained following the procedure describe for example 1, using Boc-Phe-OH instead of Boc-Leu-OH in 78% yield.
¹H-NMR (300 MHz, CDCl₃): 1.24 (m, 9H), 1.43 (s, 9H), 2.26 (m, 2H), 2.65 (m, 2H), 3.04 (m, 2H), 3.15 (m, 2H), 4.11 (m, 6H), 4.28 (m, 1H), 5.03 (br, 1H), 6.37 (bs, 1H), 7.30 (m, 5H).

### Step 2:

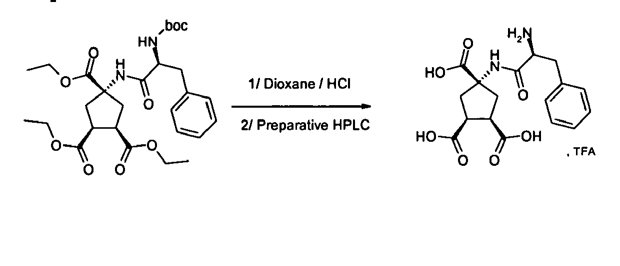

Similar deprotection conditions as the ones used to deprotect the L-Leu-ACPT-I in Example 1 were used to obtain L-Phe-ACPT-I as a trifluoroacetic acid salt after a preparative HPLC (condition B).
¹H-NMR (400 MHz, D₂O): 1.97 (m, 1H), 2.14 (m, 1H), 2.25 (m, 2H), 2.48 (dd, 1H, J=13.8, 9.5 Hz), 2.79 (q, 1H, J=8.7 Hz), 2.98 (dd, 1H, J=13.3, 10.5 Hz), 3.25 (dd, 1H, J=13.3, 5.9 Hz), 4.13 (dd, 1H, J=10.4, 5.9 Hz), 7.21-7.38 (m, 5H, Ar).
M/Z (M+H)⁺= 365.

### Example 3:

### (1S, 3R, 4S)-1-{[(2'S)-'2-aminopropanoyl]amino}cyclopentane-1,3,4-tricarboxylic acid (L -Ala ACPT-I):

### Step 1:

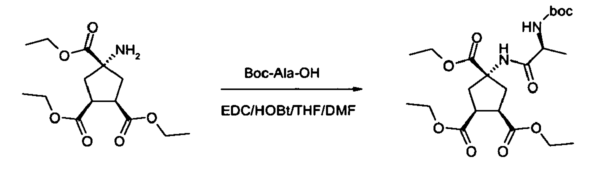

The L-Ala-ACPT-I was obtained following the procedure describe for example 1, using Boc-Ala-OH instead of Boc-Leu-OH in 82 % yield.
¹H-NMR (300 MHz, CDCl₃): 1.24 (m, 9H), 1.34 (d, 3H, J=6.9 Hz), 1.46 (s, 9H), 2.40 (m, 2H), 2.71 (m, 2H), 3.39 (m, 2H), 4.14 (m, 7H), 4.88 (br, 1H), 6.90 (bs, 1H).

### Step 2:

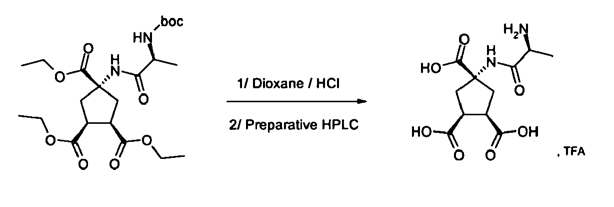

Similar deprotection conditions as the ones used to deprotect L-Leu-ACPT-I in Example 1 were used to obtain L-Ala-ACPT-I as a trifluoroacetic acid salt after a preparative HPLC (condition A).
¹H-NMR (400 MHz, D₂O): 1.50 (d, 3H, J=7.1 Hz), 2.40 (m, 2H), 2.66 (m, 2H), 3.39 (m, 2H), 4.03 (q, 1H, J=7.1 Hz).
M/Z (M+H)⁺= 289.

### Example 4:

### (1S, 3R, 4S)-1-{[(2 'S)- '2-amino-3'-methylbutanoyl]amino}cyclopentane-1,3,4-tricarboxylic acid (L-Val-ACPT-I):

### Step 1:

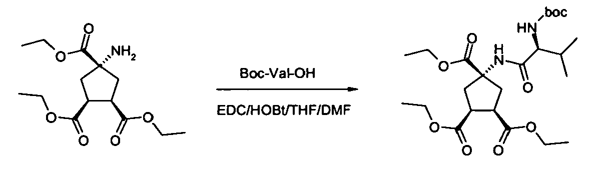

The L-Val-ACPT-I was obtained following the procedure describe for example 1, using Boc-Val-OH instead of Boc-Leu-OH in 61 % yield.
¹H-NMR (300 MHz, CDCl₃): 0.94 (d, 3H, J=6.9 Hz), 0.98 (d, 3H, J=6.6 Hz), 1.25 (t, 9H, J=7.3 Hz), 1.46 (s, 9H), 2.15 (m, 1H), 2.42 (m, 2H), 2.73 (m, 2H), 3.41 (m, 2H), 3.82 (dd, 1H, J=8.2, 8.5 Hz), 4.14 (m, 6H), 4.94 (br, 1H), 6.59 (bs, 1H).

### Step 2:

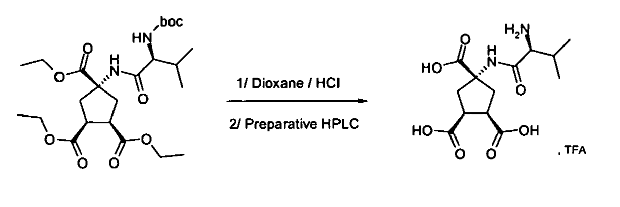

Similar deprotection conditions as the ones used to deprotect L-Leu-ACPT-I in Example 1 were used to obtain the final L-Val-ACPT-I as a trifluoroacetic acid salt after a preparative HPLC (condition B).
¹H-NMR (400 MHz, D₂O): 1.01 (dd, 6H, J=6.9, 6.9 Hz), 2.19 (m, 1H), 2.40 (m, 2H), 2.66 (m, 2H), 3.38 (m, 2H), 3.76 (d, 1H, J=5.9 Hz).
M/Z (M+H)⁺= 317.

### Example 5

### (1S, 3R, 4S)-1-{[(2'S)-2'-amino-3'-methylpentanoyl]amino}cyclopentane-1,3,4-tricarboxylic acid (L-Ile-ACPT-I):

### Step 1:

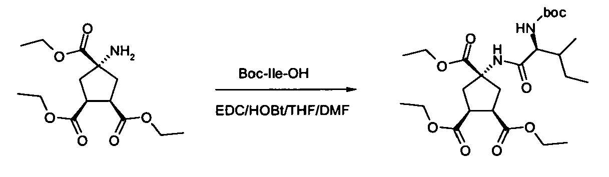

The L-Ile-ACPT-I was obtained following the procedure describe for example 1, using Boc-Ile-OH instead of Boc-Leu-OH in 78% yield.
¹H-NMR (300 MHz, CDCl₃): 0.94 (m, 6H), 1.10 (m, 2H), 1.25 (m, 9H), 1.88 (m, 1H), 2.41 (m, 2H), 2.73 (m, 2H), 3.41 (m, 2H), 3.86 (dd, 1H, J=8.6, 6.6 Hz), 4.14 (m, 6H), 4.94 (br,1H), 6.61 (s, 1H).

### Step 2:

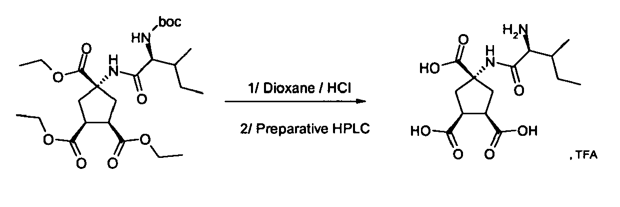

Similar deprotection conditions as the ones used to deprotect L-Leu-ACPT-I in Example 1 were used to obtain L-Ile-ACPT-Las a trifluoroacetic acid salt after a preparative HPLC (condition B).
¹H-NMR (400 MHz, D₂O): 0.87 (t, 3H, J=7.4 Hz), 0.96 (d, 3H, J=7.0 Hz), 1.16 (m, 1H), 1.46 (m, 1H), 1.91 (m, 1H), 2.38 (m, 2H), 2.62 (m, 2H), 3.34 (m, 2H), 3.78 (d, 1H, J=5.6 Hz).
M/Z (M+H)⁺ = 331.

### 3) Biological Evaluations of Prodrugs of the Invention:

### 3.1 In vivo pharmacokinetic evaluation

ACPT-I and prodrugs corresponding to examples 1, 3 and 4 were administered *per os* (*po*) at 30 mg/kg to rats. In parallel, ACPT-I was also administered intravenously (iv) at 10 mg/kg. Volume of administration is 2.0 ml/kg. Blood samples (600-700µl) were collected at time ranging from 15 minutes to 18 hours for the *po* mode of administration and from 5 minutes to 18 hours for the iv mode of administration in ice-cold tubes containing lithium-heparin and mixed gently. Immediately, 4 µl of phenylmethylsulfonyl fluorid (from a solution made of 174mg of phenylmethylsulfonyl fluorid in 5ml of ethanol 95%) were added (for 700µl of blood) to obtain a final concentration of 1 mM. Tubes were kept on ice and were centrifuged at 2500g for 10 min at 4°C. The plasma was harvested in two tubes with 150 µl each, which were kept frozen at -80°C until being analysed. 5 groups of animal were used, one for each tested prodrug and two for ACPT-I *po* and iv.

ACPT-I analysis: parent compound was analysed in plasma samples using a LC/MS/MS method. Concentrations are expressed in ng/ml of plasma.

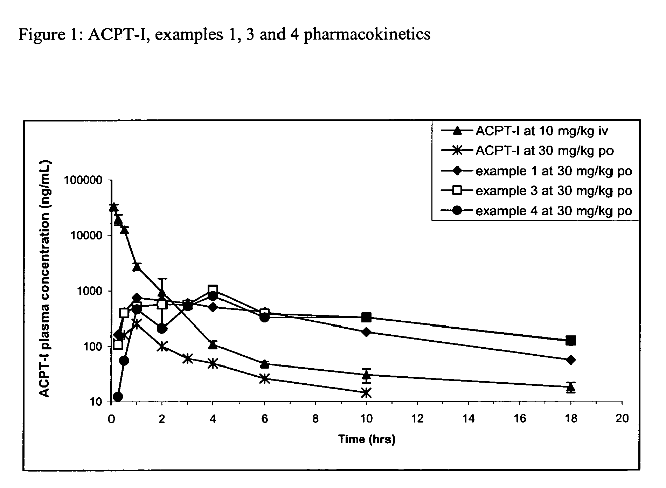

Results: figure 1 shows ACPT-I levels in plasma after oral administration of examples 1, 3, 4 and ACPT-I at 30 mg/kg and after intravenous administration of ACPT-I at 10 mg/kg.
The results show that the three tested prodrugs gave better ACPT-I exposure in plasma than the parent compound itself after oral administration. Oral bioavailabilities calculated in this experiment were:
- 1.14% for ACPT-I
- 10.40% for example 1 (L-Leu prodrug)
- 15.04% for example 3 (L-Ala prodrug)
- 12.36% for example 4 (L-Val prodrug)

### 3.2 Catalepsy model of Parkinson's disease in mice: haldol-induced catalepsy

The compounds of the invention were evaluated in a model of Parkinson's disease, the haldol-induced catalepsy model in mice.
Catalepsy was assessed using the bar test in mice submitted to acute administration of the dopamine D2 receptor antagonist haloperidol (1mg/kg, intraperitoneally or *ip).* Mice (male RjOrl: Swiss mice, weighing 30 - 35 g at the beginning of the experiment) placed in group of 5 in Plexiglas cages, were injected with haloperidol (1 mg/kg ip). Within 15 min after haloperidol administration, mice were calm and showed slow spontaneous activity. The catalepsy response of one mouse was measured as the time the animal maintained an imposed posture with both forelimbs placed on a horizontal 0.9cm-diameter wire bar suspended 4cm above a platform. The end point of catalepsy was considered to occur when both forelimbs were removed from the bar, the mouse climbed onto the bar or if the animal moved its head in an exploratory manner. A cutoff time of 180 seconds was applied. The degree of catalepsy was scored 60 minutes after haloperidol administration and continued at 30 or 60 minutes intervals for a total of 240-300 minutes. Between measurements, the animals were returned to their home cages. This procedure was adapted from the literature: *(e.g.* Pires et al., Braz J Med and Biol Res 38, 1867-1872, 2005*;* Shiozaki et al., Psychopharmacology 147, 90-95, 1999*).* Parent compound and corresponding vehicle, given either intraperitoneally (*ip*) or orally (*po*), were administered 30 minutes after haldol administration. Prodrugs and corresponding vehicle were injected 4h before haldol administration. Prodrug dose is expressed in equivalent of parent compound.

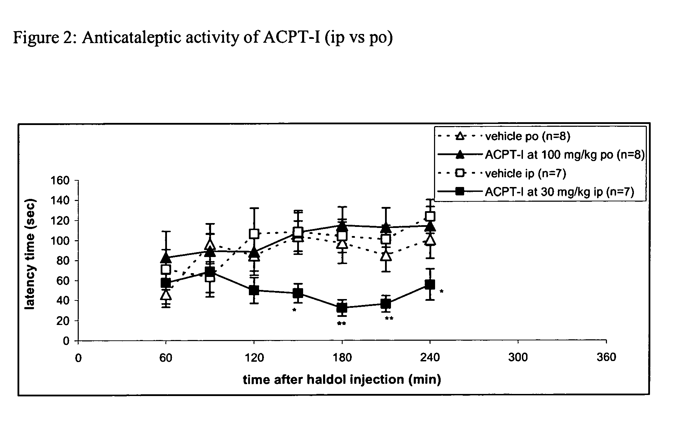

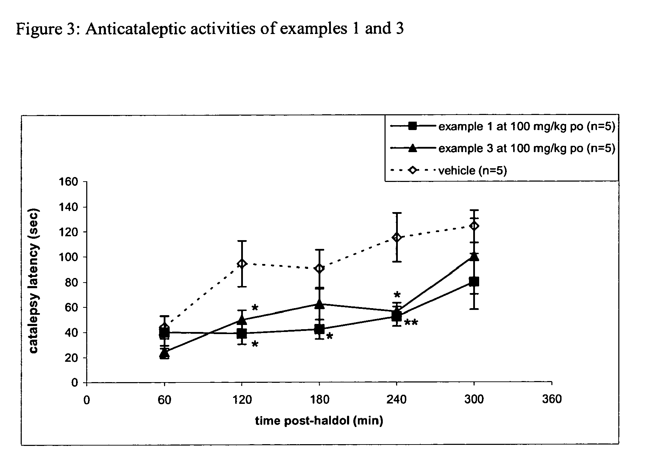

Data analysis: the figures 2 and 3 show the mean time of latency spent on the bar for each group of animals. At each time-point, the anti-cataleptic effects of parent compound and prodrugs were compared to vehicle-treated groups using 1-factor ANOVA test followed by the Dunnett's test. Datapoints marked with "*" in figures 3 or 4 indicates p<0.05 ; "**" indicates p<0.01.

Results: parent compound 100mg/kg showed no anticataleptic activity after oral administration whereas it significantly reduced catalepsy after *ip* administration of a 30mg/kg dose (Figure 2). On the opposite, L-Ala and L-Leu prodrugs orally administered at 100mg/kg significantly reduced the haldol-induced cataleptic response to the same extent as the parent compound at 30mg/kg *ip* (Figure 3).

## Claims

1. A compound of formula (I) 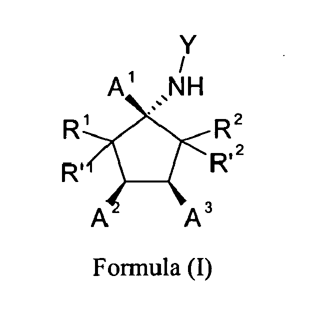 wherein
R¹, R'¹, R² and R'² are independently selected from hydrogen, optionally substituted alkyl, aryl, fluorine or hydroxy;
A¹, A² and A³ are independently selected from -COOH, its salts, esters or amides;
Y represents a -(W)ₙ-H group with
W represents an amino-acyl group corresponding to a natural or non-natural amino-acid; and
n represents an integer comprised between 1 and 10.

2. The compound of claim 1, wherein A¹, A² and A³ are -COOH.

3. The compound of claim 1 or 2, wherein R¹, R'¹, R² and R'² are hydrogen.

4. The compound of claims 1 to 3, wherein n is 1 or 2.

5. The compound of any of claims 1 to 4, wherein W is an amino-acyl group corresponding to a natural amino-acid.

6. The compound of claim 5, wherein W is an amino-acyl group corresponding to a neutral natural amino-acid.

7. The compound of claim 6, wherein the neutral natural amino-acid is selected from L-Leu, L-Ala, L-Val, L-Ile, L-Phe, Gly, L-Met, L-Ser, L-Asn, L-Gln, L-Tyr, L-Cys, L-Thr, L-Trp and L-Pro.

8. The compound of claim 7, wherein the neutral natural amino-acid is selected from L-Leu, L-Ala, or L-Val.

9. A pharmaceutical composition comprising the compound of any of claims 1 to 8 together with a pharmaceutically acceptable excipient and/or carrier.

10. The pharmaceutical composition of claim 9 which is adapted for oral or nasal administration.

11. The compound of any of claims 1 to 8 as a medicament.

12. The compound of any of claims 1 to 8 for the treatment of an indication selected from disorders of the central nervous system, neurodegenerative diseases or memory disorders.

13. The compound according to claim 12, wherein the neurodegenerative disease is Parkinson's disease, Alzheimer's disease or Huntington chorea.

14. The compound according to claim 12, wherein the disorder of the central nervous system is anxiety, schizophrenia or drug addiction.

15. A method for the preparation of a compound of any of claims 1 to 8, comprising the steps of reacting a compound of formula (II) 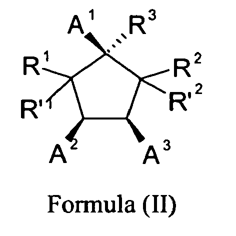 wherein R¹, R'¹, R², R'², A¹, A² and A³ are as defined for the compounds of formula (I) and R³ represents an amino group, with a natural or a non natural amino-acid or a peptide having at least one free carboxylic acid function under conditions where an amide bond is formed between the reactants.

16. The method of claim 15, wherein the compound of formula (II) is prepared by a process comprising the step of hydrogenation of a compound of formula (III) 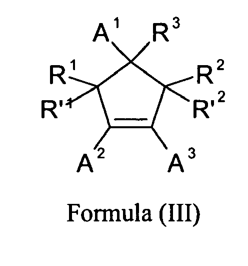 wherein R¹, R'¹, R², R'², A¹, A² and A³ are as defined for the compound of formula (I) in any of claims 1 to 3 and R³ represents an amino group or its protected form;
in the presence of a hydrogenation catalyst.
